# EUROPÄISCHE PATENTSCHRIFT

(11) **EP 3 673 878 B1**
(45) Veröffentlichungstag und Bekanntmachung des Hinweises auf die Patenterteilung: **13.10.2021**
(21) Anmeldenummer: 19204488.1
(22) Anmeldetag: 22.10.2019
(51) Int. Cl.: A61F 13/06, A61F 13/10, D04B 1/26, D04B 1/28

(54) **GESTRICKTER KOMPRESSIONSARTIKEL**
KNITTED COMPRESSION ARTICLE
ARTICLE DE CONTENTION TRICOTÉ

(30) Priorität: 05.12.2018 DE 102018130932
(43) Veröffentlichungstag der Anmeldung: 01.07.2020
(73) Patentinhaber: Julius Zorn GmbH, 86551 Aichach (DE)
(72) Erfinder: Lechner, Siegfried, 86529 Schrobenhausen (DE)
(74) Vertreter: Charrier Rapp & Liebau

(56) Entgegenhaltungen:
- WO-A1-2017/168095
- GB-A- 835 541
- JP-A- H07 252 701

## Beschreibung

Die Erfindung betrifft einen gestrickten Kompressionsartikel nach dem Oberbegriff des Anspruchs 1 sowie dessen Verwendung und ein Verfahren zur Herstellung des Kompressionsartikels.

Zur Behandlung von Narben, die bspw. durch Verbrennungen und Verbrühungen entstanden sind, werden regelmäßig Kompressionsartikel wie Kompressionsbandagen, Kompressionsstrümpfe oder Kompressionshandschuhe eingesetzt, welche einen gleichmäßigen Druck auf die betroffenen Körperstellen ausüben und dadurch wuchernde Narben (Keloide) oder Narbenkontrakturen unterbinden können. Die zur Kompressionstherapie von Narben eingesetzten Kompressionsartikel üben einen therapeutisch wirksamen Druck auf die Narbe aus, wodurch ein ungeordnetes, übermäßiges oder vermindertes Wachstum von Körpergewebe, welches zu wuchernden Narben oder zu Narbenkontrakturen führen kann, verhindert werden soll. Weiterhin wird durch die Kompressionstherapie das Narbengewebe weicher, rötliches Narbengewebe verblasst und die Narbendicke kann verringert werden.

Bei Verbrühungen oder Verbrennungen an Händen und Füßen kann es im Zwischenraum zwischen benachbarten Fingern oder Zehen zu Narbenwucherungen kommen, die zur Ausbildung von sogenannten "Schwimmhäuten" führen können. Um derartige Narbenwucherungen in den Finger- oder Zehenzwischenräumen zu unterbinden werden beispielsweise in der Narben-Kompressionstherapie von Händen Kompressionshandschuhe eingesetzt, die über sogenannte Fingerspreizer verfügen. Diese Fingerspreizer sind dabei zwischen benachbarten Fingern des Handschuhs in Form von dicken, wulstförmigen Nähten ausgebildet. Die Nähte, welche die Fingerspreizer ausbilden, werden dabei nach der Herstellung des Kompressionshandschuhs, der beispielsweise in einem Rundstrickverfahren auf einer Rundstrickmaschine gestrickt werden kann, zwischen benachbarten Fingern des Handschuhs auf dessen Außenseite aufgenäht. So können beispielsweise zwischen den Fingern 2/3, 3/4 und/oder 4/5 entsprechende Nähte zur Ausbildung von Fingerspreizern aufgenäht werden. Es ist jedoch auch möglich, Fingerspreizer nur für einen oder zwei Fingerzwischenräume vorzusehen. Die zwischen benachbarten Fingern des Kompressionshandschuhs angenähten Spreizelemente halten die Finger eines den Kompressionshandschuh tragenden Patienten einerseits auf Abstand zueinander und üben - im Vergleich zu dem vom Kompressionshandschuh auf die Hand ausgeübten Kompressionsdruck - einen erhöhten Druck auf die Fingerzwischenräume aus. Durch den zusätzlichen Druck, der von den Spreizelementen auf die Fingerzwischenräume ausgeübt wird, wird die Ausbildung von Gewebewucherungen und dadurch die Bildung von "Schwimmhäuten" in den Fingerzwischenräume unterbunden oder zumindest unterdrückt.

Die Herstellung von gestrickten Kompressionshandschuhen mit aufgenähten Spreizelementen zwischen benachbarten Fingern des Kompressionshandschuhs erweist sich jedoch als aufwändig, da die Spreizelemente nach dem Stricken des Handschuhs in einem separaten Herstellungsschritt von Hand oder maschinell aufgenäht werden müssen. Weiterhin vermindern die aus relativ dicken Nähten hergestellten Spreizelemente den Tragekomfort.

Aus der WO 2017/168095 A1 und der GB 835541-A sind Verfahren zum Stricken von nahtlosen Handschuhen bzw. von nahtlosen schlauchförmigen Strickteilen bekannt, in denen die Finger der Handschuhe durch sogenanntes Fingerkreuzen miteinander verbunden werden. Die offenbarten Strickverfahren werden dabei auf einer Flachstrickmaschine mit einem vorderen und einem hinteren Nadelbett durchgeführt, wobei der gestrickte Handschuh eine auf dem vorderen Nadelbett gestrickte vordere Lage und eine auf dem hinteren Nadelbett gestrickte hintere Lage aufweist. Die JPH 07252701-A zeigt Socken mit mehreren Aufnahmen für die Zehen eines Trägers, wobei benachbarte Aufnahmen mittels Verbindungsteile miteinander verbunden sind.

Hiervon ausgehend liegt der Erfindung die Aufgabe zugrunde, einen gestrickten Kompressionsartikel, wie z. B. einen Kompressionshandschuh oder -füßling für die Kompressionstherapie von Händen oder Füßen bereitzustellen, der einfacher und insbesondere in einem durchgehenden Strickvorgang ohne nachgeschaltete Näharbeiten herstellbar ist, und über Spreizelemente verfügt, die in den Zwischenräumen zwischen benachbarten Fingern oder Zehen eines Patienten einen erhöhten Kompressionsdruck ausüben, um Narbenkeloide und insbesondere die Ausbildung sogenannter "Schwimmhäute" und/oder Narbenkontraktionen zu unterdrücken sowie einen besseren Tragekomfort aufweist.

Diese Aufgaben werden mit einem gestrickten Kompressionsartikel mit den Merkmalen des Anspruchs 1 gelöst. Bevorzugte Ausführungsformen des erfindungsgemäßen Kompressionsartikels sowie mögliche Verwendungen und ein Verfahren zu seiner Herstellung ergeben sich aus den abhängigen Ansprüchen.

Bei dem erfindungsgemäßen Kompressionsartikel handelt es sich um einen gestrickten Kompressionsartikel, beispielsweise in der Form eines Handschuhs, eines Strumpfs oder eines Füßlings, mit einer Mehrzahl von schlauch- oder taschenförmigen Aufnahmen für wenigstens einen Finger oder einen Zeh eines Trägers des Kompressionsartikels, wobei mindestens zwischen zwei benachbarten Aufnahmen ein Spreizelement angeordnet ist. Dabei weist erfindungsgemäß jede der Aufnahmen eine vordere Lage und eine hintere Lage eines Gestricks auf, wobei die vordere Lage und die hintere Lage nahtlos miteinander verstrickt sind, um die schlauch- oder taschenförmige Aufnahme auszubilden, wobei das oder jedes Spreizelement, das zwischen zwei benachbarten Aufnahmen angeordnet ist, beim Strickvorgang durch stricktechnisches Verbinden der vorderen Lage und der hinteren Lage ausgebildet ist.

Durch die stricktechnische Verbindung der vorderen Lage und der hinteren Lage von benachbarten Aufnahmen des erfindungsgemäßen Kompressionsartikels können die Spreizelemente beim Stricken des Kompressionsartikels eingestrickt werden. Dadurch ist es nach Beendigung des Strickvorgangs nicht mehr erforderlich die Spreizelemente nachträglich durch Aufnähen von Nähten auf der Außenseite des Kompressionsartikels zwischen benachbarten Aufnahmen zu fertigen. Der erfindungsgemäße Kompressionsartikel kann deshalb einfacher und schneller und damit kostengünstiger hergestellt werden. Desweiteren weisen erfindungsgemäße Kompressionsartikel einen erhöhten Tragekomfort auf, weil sie nicht über nachträglich angebrachte Nähte verfügen und es hat sich gezeigt, dass erfindungsgemäße Kompressionsartikel einfacher und schneller an einer Hand oder einem Fuß eines Patienten anlegbar sind, da die eingestrickten Spreizelemente benachbarte Finger oder Zehen beim Anziehen des Kompressionsartikels auf Abstand zueinander halten.

Die beim erfindungsgemäßen Kompressionsartikel beim Strickvorgang eingestrickten Spreizelemente weisen keine Nähte auf, weshalb der erfindungsgemäße Kompressionsartikel insgesamt nahtlos ausgebildet werden kann. Eine nahtlose Ausbildung des Kompressionsartikels ermöglicht einen besseren Tragekomfort sowie eine gleichmäßigere Verteilung des vom Kompressionsartikel auf die Hand oder den Fuß des Trägers ausgeübten Kompressionsdrucks.

Eine nahtlose Fertigung eines aus einer vorderen und einer hinteren Lage bestehenden Kompressionsartikels mit mehreren Aufnahmen für Finger oder Zehen eines Trägers des Kompressionsartikels kann beispielsweise auf einer Flachstrickmaschine mit zwei gegenüberliegenden Nadelbetten, nämlich einem vorderen und einem diesem gegenüberliegenden hinteren Nadelbett, gestrickt werden.

Bei der Herstellung des erfindungsgemäßen Kompressionsartikels auf einer Flachstrickmaschine mit zwei gegenüberliegenden Nadelbetten wird zur Ausbildung des Kompressionsartikels ein schlauchförmiger Grundkörper mit einer vorderen Lage, die auf dem vorderen Nadelbett der Flachstrickmaschine gestrickt wird, und einer hinteren Lage, die auf dem hinteren Nadelbett der Flachstrickmaschine gestrickt wird, ausgebildet, wobei die vordere und die hintere Lage des schlauchförmigen Grundkörpers nahtlos miteinander verstrickt sind. In entsprechender Weise werden auch Aufnahmen für die Finger oder die Zehen eines Trägers des Kompressionsartikels nahtlos auf einer Flachstrickmaschine gestrickt, wobei auch die Aufnahmen jeweils eine vordere und eine hintere Lage aufweisen, die auf dem vorderen bzw. dem hinteren Nadelbett der Flachstrickmaschine gestrickt und nahtlos miteinander verbunden werden, um die schlauch- oder taschenförmige Aufnahme auszubilden. Für die Ausbildung von Spreizelementen zwischen benachbarten Aufnahmen werden dabei die vordere Lage und die hintere Lage der zueinander benachbarten Aufnahmen stricktechnisch so miteinander verbunden, dass sich im Zwischenraum zwischen den benachbarten Aufnahmen ein flaches Spreizelement ausbildet. Das flache Spreizelement zwischen zwei benachbarten Aufnahmen kann sich dabei nur über eine einzige Masche oder auch über eine Mehrzahl von Maschen des Gestricks des gestrickten Kompressionsartikels erstrecken. Zweckmäßig erfolgt das stricktechnische Verbinden der vorderen und der hinteren Lage der beiden zueinander benachbarten Aufnahmen durch Verkreuzung der vorderen Lage und der hinteren Lage.

Der erfindungsgemäße Kompressionsartikel umfasst damit einen gestrickten Grundkörper und daran angestrickte schlauch- oder taschenförmige Aufnahmen für die Aufnahme eines oder mehrerer Finger oder eines oder mehrerer Zehen, wobei bevorzugt der Grundkörper des Kompressionsartikels und die Aufnahmen aus demselben Grundgestrick mit einer beim Tragen des Kompressionsartikels der Körperextremität (beispielsweise einer Hand oder einem Fuß eines Trägers) zugewandten Innenseite und einer dieser gegenüberliegenden Außenseite gestrickt werden. Das Grundgestrick ist dabei zweckmäßig entlang einer sich zumindest im Wesentlichen in Längsrichtung der Aufnahmen erstreckenden Maschenstäbchenrichtung gestrickt und weist quer zur Maschenstäbchenrichtung verlaufende Maschenreihen auf. Das Grundgestrick ist dabei aus einem maschebildenden Strickfaden gestrickt, der elastisch oder unelastisch sein kann. In das Grundgestrick ist bevorzugt ein kompressionsgebender elastischer Schussfaden eingebunden oder eingelegt. Bevorzugt ist in jeder Maschenreihe des Grundgestricks ein Schussfaden vorhanden. Es ist jedoch auch möglich, beispielsweise für die Ausbildung eines Kompressionsartikels mit einer geringeren Kompressionswirkung, nur in jeder zweiten oder jeder dritten Maschenreihe etc., einen Schussfaden vorzusehen.

In den Bereichen des Grundgestricks, in denen Spreizelemente ausgebildet sind, also zwischen zwei benachbarten Aufnahmen, liegt der Schussfaden bevorzugt flott in dem Grundgestrick. Die zwischen den beiden benachbarten Aufnahmen ausgebildeten Spreizelemente halten dadurch die in den Aufnahmen aufgenommenen Finger oder Zehen eines Trägers des Kompressionsartikels auf Abstand zueinander, wodurch die Ausbildung von Narbenwucherungen oder sogenannten "Schwimmhäuten" unterdrückt wird. Weiterhin kann der Kompressionsartikel dadurch leichter an einer Hand oder einem Fuß durch Einschieben der Finger bzw. Zehen in die dafür vorgesehenen Aufnahmen angelegt werden, da die zwischen benachbarten Aufnahmen angeordneten Spreizelemente die Finger oder Zehen des Trägers beim Anziehen leicht auseinander drücken, so dass der Träger des Kompressionsartikels leichter mit seinen Fingern bzw. Zehen in die dafür vorgesehenen Aufnahmen des Kompressionsartikels schlüpfen kann. Bei einem Zehenteil erleichtert sich das Anziehen ferner durch eine verbesserte Kraftverteilung, wenn beim Anziehen an dem Zehenteil gezogen wird. Dabei wird durch die Verbindung über die Spreizelemente die aufgewandte Kraft auf den benachbarten Zehen mit übertragen, wodurch die Kraft besser verteilt und das Anziehen somit erleichtert wird.

Das Grundgestrick des erfindungsgemäßen Kompressionsartikels kann zweckmäßig eine Mehrzahl von rippenförmigen Erhebungen aufweisen, die zumindest im Wesentlichen parallel und in einem vorgegebenen Abstand zueinander sowie entlang einer Längsrichtung der Aufnahmen verlaufen. Die Ausbildung solcher Erhebungen, die zweckmäßig an der Außenseite über dem Grundgestrick vorstehen, erleichtert das Anziehen des Kompressionsartikels an einer Hand oder einem Fuß, da die rippenförmigen Erhebungen die Längszügigkeit des Kompressionsartikels verbessern.

Die rippenförmigen Erhebungen des Grundgestricks erstrecken sich dabei zweckmäßig über mindestens zwei Maschen, um den gewünschten Effekt zu erzielen. Die rippenförmigen Erhebungen können bspw. durch Flottungen und/oder durch Fanghenkel des Strickfadens in dem Grundgestrick ausgebildet sein.

In einer bevorzugten Ausführungsform der Erfindung ist das Grundgestrick des Kompressionsartikels ein Rechts-Links-Gestrick (RL). Für die Ausbildung des Grundgestricks des erfindungsgemäßen Kompressionsartikels können jedoch auch alle anderen gängigen Bindungsarten, beispielsweise eine 1:1-Bindung oder eine Rechts-Rechts-Bindung (RR), verwendet werden. Insbesondere bei der bevorzugten Herstellung des erfindungsgemäßen Kompressionsartikels auf einer Flachstrickmaschine können grundsätzlich alle auf einer Flachstrickmaschine herstellbare Gestrickbindungen verwendet werden, um das Grundgestrick auszubilden und ggf. einen elastischen Schussfaden darin einzubinden.

Der erfindungsgemäße Kompressionsartikel kann neben dem bevorzugten Einsatz in der Narbentherapie oder der Behandlung von Verbrennungen und Verbrühungen an Händen und Füßen weiterhin auch im Bereich der plastisch-chirurgischen Rekonstruktion einer Hand oder eines Fußes und insbesondere zur postoperativen Kompressionstherapie im Bereich der plastisch-rekonstruktiven Chirurgie verwendet werden.

Diese und weitere Merkmale sowie Vorteile und Wirkungen des erfindungsgemäßen Kompressionsartikels ergeben sich aus den nachfolgenden unter Bezugnahme auf die begleitenden Zeichnungen näher beschriebenen Ausführungsbeispielen. Die Zeichnungen zeigen:
- **Fig. 1:**: Schematische Darstellung eines erfindungsgemäßen Kompressionsartikels in Form eines an einer Hand eines Trägers angelegten Kompressionshandschuhs;
- **Fig. 2:**: Schematische Darstellung eines erfindungsgemäßen Kompressionsartikels in Form eines an einen Fuß eines Trägers angelegten Zehenteils;
- **Fig. 3:**: Verschiedene Ausführungsbespiele für Gestrickbindungen des Grundgestricks von erfindungsgemäßen Kompressionsartikeln (Fig. 3a bis 3c) mit einer zugehörigen Legende der in den Gestrickbildern der Fig. 3a bis 3c verwendeten Symbole (Fig. 3d).

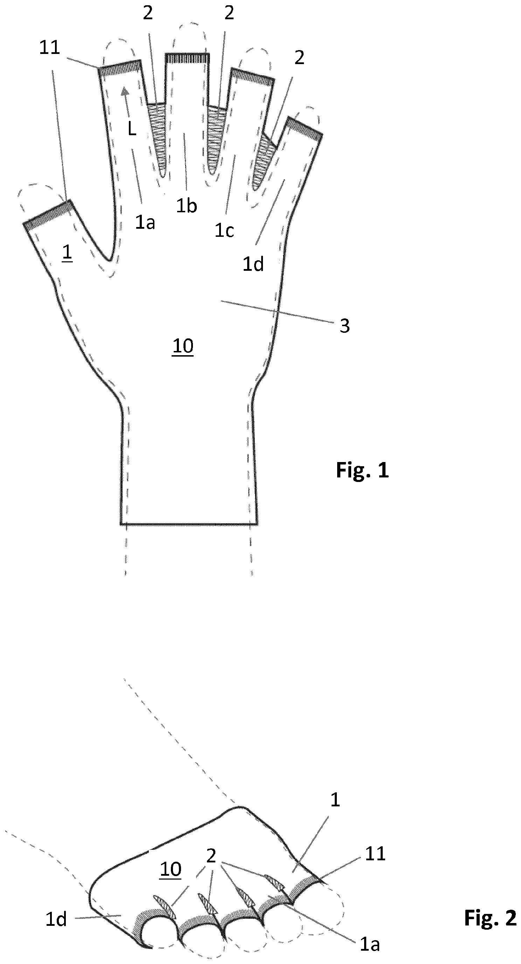

In Fig. 1 ist ein erstes Ausführungsbeispiel für einen gestrickten Kompressionsartikel gemäß der Erfindung in Form eines Kompressionshandschuhs in einer Draufsicht dargestellt. Der Kompressionshandschuh ist dabei an der Hand eines Trägers, die gestrichelt dargestellt ist, angelegt. Der Kompressionshandschuh weist einen schlauchförmigen Grundkörper 10 auf, an den sich insgesamt fünf Aufnahmen 1, 1a - 1d anschließen. Die Aufnahmen 1, 1a - 1d sind jeweils schlauchförmig ausgebildet und dienen der Aufnahme der fünf Finger der Hand des Trägers, wobei eine erste Aufnahme 1 zur Aufnahme des Daumens und die übrigen vier Aufnahmen 1a - 1d zur Aufnahme der übrigen vier Finger der Hand dienen. Die im Folgenden auch gemeinsam mit Bezugszeichen 1 bezeichneten Aufnahmen 1 und 1a bis 1d sind dabei an ihrem vorderen (distalen) Ende offen, so dass - wie aus Fig. 1 ersichtlich - die Fingerkuppen aus den offenen Enden der Aufnahmen 1, 1a - 1d herausragen. Am distalen Ende der Aufnahmen 1 ist jeweils ein Abschluss in Form eines Bündchens 11 angestrickt. Es ist jedoch auch möglich, die Aufnahmen 1 an ihrem distalen Ende mit einer domförmigen Kappe zu verschließen, so dass sich eine geschlossene Aufnahme für die Finger der Hand ergibt.

Wie aus Fig. 1 ersichtlich sind zwischen den zueinander benachbarten Aufnahmen 1a und 1b, 1b und 1c sowie 1c und 1d Spreizelemente 2 vorgesehen. Die Spreizelemente 2 erstrecken sich dabei über mindestens 1/3, besonders bevorzugt über mindestens die Hälfte der Länge der Aufnahmen 1a bis 1d (also bspw. etwa über die halbe Ausdehnung der Aufnahmen in deren Längsrichtung L, wie in Figur 1 gezeigt). Dadurch können die beiden benachbarten Aufnahmen 1a, 1b; 1b, 1c; 1c, 1d von dem dazwischenliegenden Spreizelement 2 ausreichend auf Abstand gehalten werden.

Der in Fig. 1 gezeigte Kompressionshandschuh ist nahtlos in einem Stück, also einschließlich der Spreizelemente 2, auf einer Flachstrickmaschine mit einem vorderen und einem diesem gegenüberliegenden hinteren Nadelbett gestrickt. Dabei ist der Grundkörper 10 und die sich daran anschließenden Aufnahmen 1 jeweils aus einem Grundgestrick 3 gestrickt, welches eine vordere Lage v und eine nahtlos mit dieser verbundenen hintere Lage h umfasst. Die vordere Lage v des Grundgestricks 3 ist dabei auf dem vorderen Nadelbett der Flachstrickmaschine und die hintere Lage h auf dem hinteren Nadelbett gestrickt. Durch die stricktechnische, nahtlose Verbindung der vorderen Lage v und der hinteren Lage h ergibt sich eine schlauch- bzw. taschenförmige Ausbildung des Grundkörpers 10 und der Aufnahmen 1, 1a - 1d.

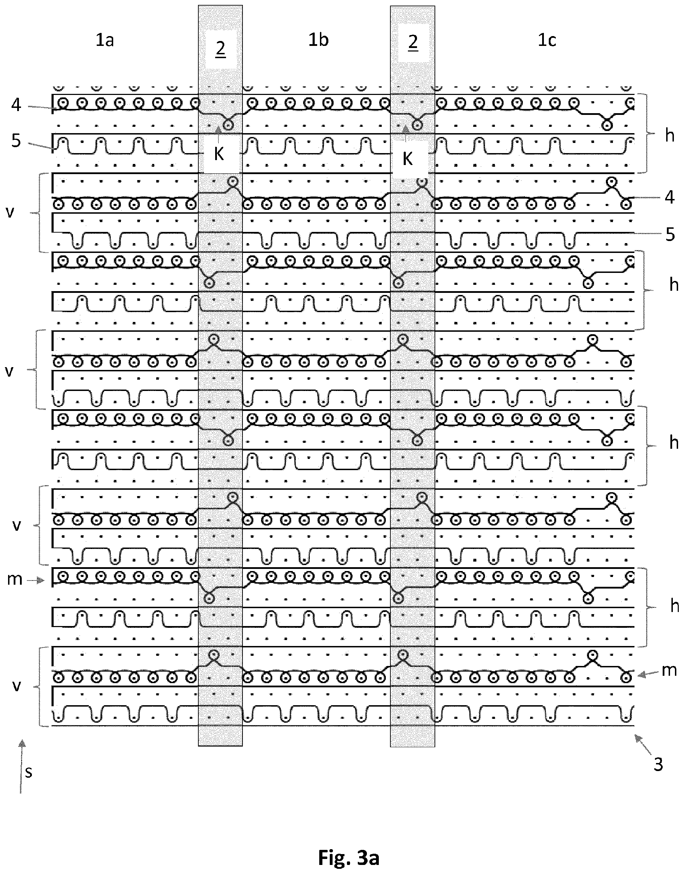

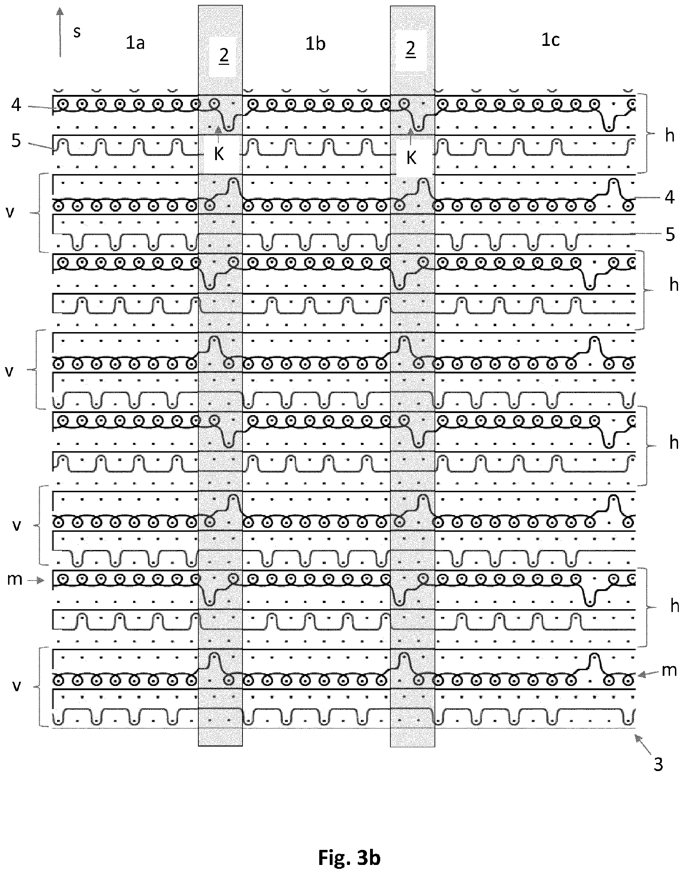

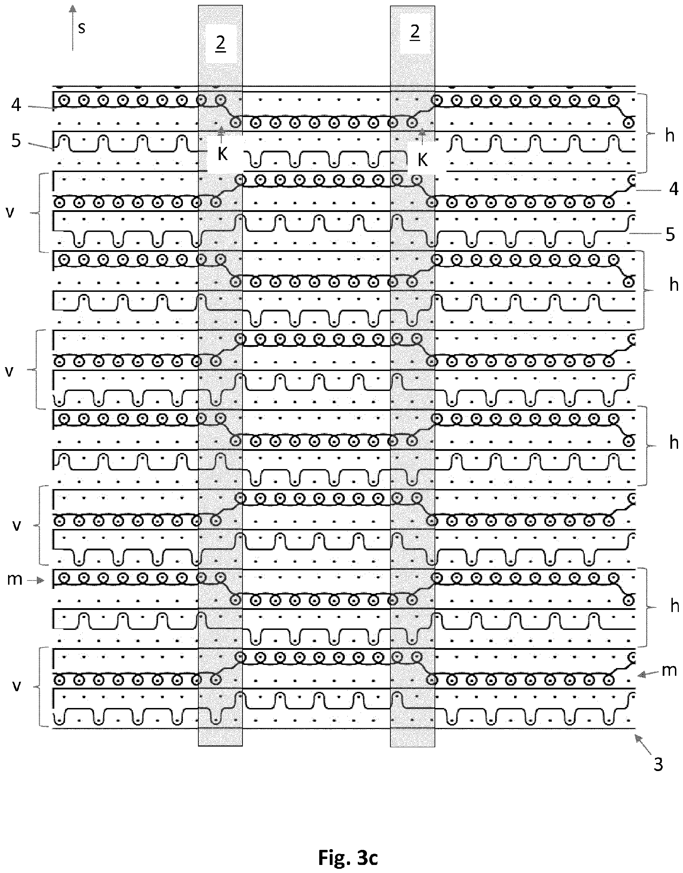

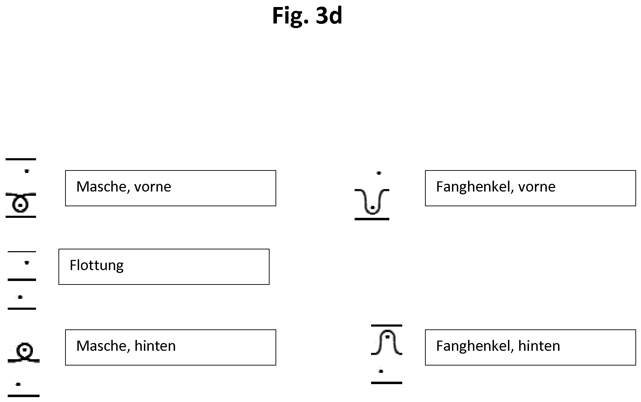

Die in den Zwischenräumen zwischen dem zweiten und dritten Finger (1a, 1b), zwischen dem dritten und vierten Finger (1b, 1c) sowie dem vierten und fünften Finger (1c, 1d) ausgebildeten Spreizelemente 2 sind ebenfalls beim Stricken des Kompressionsartikels auf einer Flachstrickmaschine in das Grundgestrick 3 eingestrickt, wobei jedes Spreizelement 2 durch stricktechnisches Verbinden der vorderen Lage v und der hinteren Lage h der zueinander benachbarten Aufnahmen 1a, 1b; 1b, 1c; 1c, 1d und insbesondere durch Verkreuzung der vorderen Lage v und der hinteren Lage h gebildet ist. Die Spreizelemente 2 erstrecken sich dabei in Richtung der Maschenreihen m über mindestens zwei Maschen (Figur 3).

Zur Erzeugung einer Kompressionswirkung ist in dem Grundgestrick 3, aus dem der Grundkörper 10 und die Aufnahmen 1 gestrickt sind, ein elastischer Schussfaden eingebunden bzw. eingelegt. Der Schussfaden ist dabei mit einer Vorspannung im Grundgestrick 2 eingebunden bzw. eingelegt. Als Schussfaden kann beispielsweise ein Umwindegarn mit einem hochelastischen Kernfaden oder ein Elastan- oder Gummifaden verwendet werden, wobei der Kernfaden bzw. der Elastan- oder Gummifaden bevorzugt eine Dicke im Bereich von 200 bis 1500 dtex aufweist. Das Grundgestrick 3 ist aus einem elastischen oder unelastischen Strickfaden gestrickt. Bei Verwendung eines elastischen Strickfadens kann beispielsweise ein Umwindegarn mit einem elastischen Kernfaden eingesetzt werden. Der Schussfaden weißt dabei zweckmäßig eine größere Dicke als der Strickfaden auf.

Für die Ausbildung des Grundgestricks des Grundkörpers 10 und der Aufnahmen 1 können alle gängigen Gestrickbindungen verwendet werden. Zweckmäßig ist das Grundgestrick ein Rechts-Links-Gestrick und der Schussfaden ist in dem Grundgestrick 1:1 versetzt hinterlegt.

In Fig. 2 ist ein zweites Ausführungsbeispiel eines erfindungsgemäßen Kompressionsartikels in Form eines Zehenteils mit einem schlauchförmigen Grundkörper 10 und sich daran anschließenden Aufnahmen 1, 1a - 1d gezeigt. Wie bei dem Ausführungsbeispiel von Fig. 1 ist der schlauchförmige Grundkörper 10 und die fünf Aufnahmen 1, 1a - 1d aus einem Grundgestrick in einem einzigen Strickvorgang hergestellt, wobei das Stricken bevorzugt auf einer Flachstrickmaschine mit einem vorderen und einem hinteren Nadelbett erfolgt, auf der jeweils eine vordere Lage und eine hintere Lage des Grundkörpers 10 und der Aufnahmen 1, 1a - 1d gestrickt und nahtlos so miteinander verbunden werden, dass sich schlauch- bzw. taschenförmige Grundkörper 10 und Aufnahmen 1 ergeben. Die Aufnahmen 1 sind auch bei dem Ausführungsbeispiel der Fig. 2 an ihrem distalen Ende offen, so dass die in den Aufnahmen 1, 1a - 1d aufgenommenen Zehen eines Trägers am vorderen Ende der offenen Aufnahmen 1 herausragen.

In Fig. 3 sind verschiedene Ausführungsbeispiele für Gestrickbindungen gezeigt, mit denen erfindungsgemäße Kompressionsartikel gestrickt werden können.

Ein erstes Ausführungsbeispiel einer geeigneten Gestrickbindung ist in Fig. 3a gezeigt. Die dargestellte Gestrickbindung zeigt ein Grundgestrick 3 im Bereich der Aufnahmen 1a - 1c, wobei sich zwischen benachbarten Aufnahmen 1a, 1b; 1b, 1c Spreizelemente 2 erstrecken. Die Spreizelemente 2 sind dabei beim Stricken des Grundgestricks 3 durch stricktechnisches Verbinden einer vorderen Lage v und einer hinteren Lage h der Aufnahmen 1a, 1b und 1c ausgebildet.

Das in Fig. 3a gezeigte Grundgestrick 3 kann bspw. nahtlos auf einer Flachstrickmaschine mit einem vorderen und einem hinteren Nadelbett gestrickt werden, wobei auf dem vorderen Nadelbett eine vordere Lage v und auf dem hinteren Nadelbett eine hintere Lage h der Aufnahmen 1a - 1c gestrickt wird. Die vordere Lage v und die hintere Lage h der Aufnahmen 1a - 1c werden dabei nahtlos so miteinander verbunden, dass sich aus den beiden Lagen v, h, eine schlauch- bzw. taschenförmige Aufnahme ergibt. Die Aufnahmen 1a - 1c können dabei - wie in den Fig. 1 und 2 gezeigt - am distalen Ende offen sein und ein Bündchen 11 aufweisen.

Aus dem Gestrickbild der Fig. 3a ist die stricktechnische Ausbildung der Spreizelemente 2 beim Stricken der Aufnahmen 1 ersichtlich.

Das in Fig. 3a gezeigte Grundgestrick 3 ist aus einem elastischen oder unelastischen, maschebildenden Strickfaden 4 gestrickt. In das Grundgestrick 3 ist ein elastischer Schussfaden 5 in Richtung der Maschenreihen m des Grundgestricks 3 verlaufend eingebunden. Das Grundgestrick 3 weist eine auf dem vorderen Nadelbett der Flachstrickmaschine gestrickte vordere Lage v und eine auf dem hinteren Nadelbett der Flachstrickmaschine gestrickte hintere Lage h auf. Der Schussfaden ist bei dem in Fig. 3a gezeigten Grundgestrick 3 in jede Maschenreihe m eingebunden. Es ist jedoch auch möglich, den Schussfaden 5 nur in jeder zweiten Maschenreihe oder nur in jeder dritten Maschenreihe, usw. einzulegen.

Im Bereich der schlauchförmigen Aufnahmen 1a, 1b, 1c bildet der Strickfaden 4 Maschen, die in Maschenreihenrichtung m nebeneinander angeordnet sind und in Maschenstäbchenrichtung s maschebildend miteinander verstrickt sind. Der Schussfaden 5 bildet im Bereich der Aufnahmen 1a, 1b, 1c abwechselnd Fanghenkel und Flottungen.

Im Bereich der Spreizelemente 2 erfolgt eine Verkreuzung der vorderen Lage v und der hinteren Lage h der Aufnahmen 1a, 1b, 1c an Kreuzungsstellen K. An den Kreuzungsstellen K wird der Strickfaden 4 beim Stricken von einer Lage (beispielsweise der vorderen Lage v) auf die gegenüberliegende Lage (hintere Lage h) umgelegt, wobei der Strickfaden 4 an der Kreuzungsstelle K flott liegt und nach dem Umlegen auf die gegenüberliegende Lage dort eine Masche ausbildet. In der in Maschenreihenrichtung benachbarten Masche wird der Strickfaden 4 wieder auf die ursprüngliche Lage (vordere Lage v) umgelegt und bildet dort wieder eine Masche. Durch das Umlegen des Strickfadens 4 von einer Lage auf die gegenüberliegende Lage wird der Schlauch der schlauchförmigen Aufnahmen 1a, 1b, 1c an den Kreuzungsstellen K geschlossen, wodurch sich an den Kreuzungsstellen K ein die benachbarten Aufnahmen 1a, 1b; 1b, 1c verbindendes Spreizelement 2 ergibt. Das Spreizelement 2 hält dabei die zueinander benachbarten Aufnahmen auf Abstand zueinander und bewirkt beim Tragen des Kompressionshandschuhs an der Hand eines Trägers einen erhöhten Druck im Zwischenraum zwischen benachbarten Fingern aus.

In Fig. 3b ist ein zweites Ausführungsbeispiel einer Gestrickbindung für einen Kompressionsartikel gemäß der Erfindung im Bereich von zueinander benachbarten Aufnahmen 1a, 1b, 1c dargestellt. Wie beim Ausführungsbeispiel von Fig. 3a sind die schlauch- oder taschenförmigen Aufnahmen 1a, 1b, 1c mit den in den Zwischenräumen angeordneten Spreizelementen 2 aus einem Grundgestrick 3 gebildet, wobei das Grundgestrick 3 einen maschebildenden Strickfaden 4 und einen in das Grundgestrick in jeder Maschenreihe m eingebundenen elastischen Schussfaden 5 umfasst. Die schlauch- oder taschenförmigen Aufnahmen 1a, 1b, 1c weisen dabei eine vordere Lage v und eine nahtlos mit dieser verbundenen hinteren Lage h auf, wobei die vordere Lage v auf dem vorderen Nadelbett einer Flachstrickmaschine und die hintere Lage h auf dem hinteren Nadelbett der Flachstrickmaschine gestrickt werden kann. Die in den Zwischenräumen zwischen benachbarten Aufnahmen 1a, 1b; 1b, 1c liegenden Spreizelemente 2 sind dabei beim Stricken durch Verkreuzung der vorderen Lage v und der hinteren Lage h der Aufnahmen 1a, 1b, 1c an Kreuzungsstellen K eingestrickt. Anders als bei dem Ausführungsbeispiel von Fig. 3a ist dabei der maschebildende Strickfaden 4 beispielsweise von einer hinteren Lage h an einer Kreuzungsstelle K auf die vordere Lage v umgelegt und bildet in der vorderen Lage v einen Fanghenkel. In der in Maschenreihenrichtung m neben diesem Fanghenkel liegenden Masche des Strickfadens 4 ist wieder auf die hintere Lage h umgelegt und bildet dort eine Masche. Dadurch bildet sich im Bereich der Kreuzungsstelle K ein Spreizelement 2 aus, das sich in Maschenreihenrichtung m über zwei Maschen erstreckt.

Der Schussfaden 5 bildet dabei in Maschenreihenrichtung m abwechselnd Fang und Flottungen in dem Grundgestrick 3 aus und liegt im Bereich der Spreizelemente 2 (also an den Kreuzungsstellen K) flott im Grundgestrick.

In dem in Fig. 3c gezeigten Ausführungsbeispiel eines Grundgestricks 3 für einen erfindungsgemäßen Kompressionsartikel erfolgt im Bereich der Kreuzungsstellen K, an denen der maschebildende Strickfaden 4 von der vorderen Lage v auf die hintere Lage h (und umgekehrt) umgelegt wird, ein Wechsel der gegenüberliegenden Lagen v und h. Wie aus Fig. 3c ersichtlich, wird der maschebildende Strickfaden 4, nachdem er beispielsweise von der hinteren Lage h auf die vordere Lage v an einer Kreuzungsstelle K umgelegt worden ist, auf der vorderen Lage v in Maschenreihenrichtung m weiter als Masche gestrickt, wodurch sich eine Vertauschung der hinteren Lage h und der vorderen Lage v in zueinander benachbarten Aufnahmen 1a, 1b ergibt. Wie in den Ausführungsbeispielen der Fig. 3a und 3c werden durch das Verkreuzen des maschebildenden Strickfadens 4 an den Kreuzungsstellen K die schlauchförmige Aufnahme 1a, 1b, 1c geschlossen und zwischen benachbarten Aufnahmen 1a, 1b; 1b, 1c die Spreizelemente 2 ausgebildet.

Die Erfindung ist nicht auf die hier zeichnerisch dargestellten Ausführungsbeispiele und insbesondere nicht auf die bevorzugte Herstellung auf einer Flachstrickmaschine mit einem vorderen und einem hinteren Nadelbett beschränkt. Das Grundgestrick des erfindungsgemäßen Kompressionsartikels kann in entsprechender Weise auch als Rundgestrick auf einer Rundstrickmaschine hergestellt werden. Weiterhin können für die Herstellung des Grundgestricks 3 andere Bindungsarten eingesetzt werden. Die Form der erfindungsgemäßen Kompressionsartikel ist nicht auf die hier gezeigten Ausführungsbeispiele eines Handschuhs und eines Zehenteils beschränkt. So kann beispielsweise statt eines Zehenteils auch ein Füßling, ein Socken oder ein Strumpf mit (am distalen Ende offenen oder geschlossenen) Aufnahmen für die Zehen eines Trägers ausgeformt werden.

Weiterhin ist es möglich, in einem erfindungsgemäßen Kompressionsartikel Aufnahmen für mehr als einen Finger oder mehr als einen Zeh vorzusehen. Dies kann u. U. aus therapeutischen Gründen zweckdienlich sein, beispielsweise, wenn benachbarte Finger oder Zehen zur Stabilisierung in einer gemeinsamen Aufnahme untergebracht werden sollen.

## Patentansprüche

1. Gestrickter Kompressionsartikel mit einer Mehrzahl von schlauch- oder taschenförmigen Aufnahmen (1) für wenigstens einen Finger oder wenigstens einen Zeh eines Trägers des Kompressionsartikels, wobei mindestens zwischen zwei benachbarten Aufnahmen (1a, 1b) ein Spreizelement (2) angeordnet ist, wobei jede Aufnahme (1) eine vordere Lage (v) und eine hintere Lage (h) aufweist, wobei die vordere Lage (v) und die hintere Lage (h) nahtlos miteinander verstrickt sind, um die schlauch- oder taschenförmige Aufnahme (1) auszubilden, und dass das oder jedes Spreizelement (2) durch stricktechnisches Verbinden der vorderen Lage (v) und der hinteren Lage (h) gebildet ist, **dadurch gekennzeichnet, dass** sich das oder jedes Spreizelement (2) über mindestens 1/3 der Länge der jeweils angrenzenden Aufnahme (1) erstreckt.

2. Kompressionsartikel nach Anspruch 1, **dadurch gekennzeichnet, dass** der Kompressionsartikel insgesamt nahtlos ist, wobei die Spreizelemente (2) ohne Nähte ausgebildet sind.

3. Kompressionsartikel nach einem der voranstehenden Ansprüche, **dadurch gekennzeichnet, dass** das oder jedes Spreizelement (2) durch Verkreuzung der vorderen Lage (v) und der hinteren Lage (h) gebildet ist.

4. Kompressionsartikel nach einem der voranstehenden Ansprüche, **dadurch gekennzeichnet, dass** der Kompressionsartikel ein Grundgestrick (3) mit einer beim Tragen der Körperextremität zuwendbaren Innenseite (I) und einer dieser gegenüber liegenden Außenseite (A) aufweist, wobei das Grundgestrick (3) entlang einer sich zumindest im Wesentlichen in Längsrichtung (L) der oder jeder Aufnahme (1) erstreckenden Maschenstäbchenrichtung (s) gestrickt ist und quer zur Maschenstäbchenrichtung (s) verlaufende Maschenreihen (m) aufweist, wobei das Grundgestrick (3) ein Rechts-Links-Gestrick oder ein Rechts-Rechts-Gestrick ist und eine vordere Lage (v) sowie eine nahtlos mit der vorderen Lage (v) verbundene hintere Lage (h) umfasst, wobei die vordere Lage (v) und die hintere Lage (h) aus wenigstens einem elastischen oder unelastischen Strickfaden (4) gebildet ist.

5. Kompressionsartikel nach Anspruch 4, **dadurch gekennzeichnet, dass** ein Schussfaden (5) in das Grundgestrick (3) eingebunden oder eingelegt ist.

6. Kompressionsartikel nach einem der voranstehenden Ansprüche, **dadurch gekennzeichnet, dass** sich die Spreizelemente (2) in Richtung der Maschenreihen (m) über mindestens zwei oder drei Maschen erstrecken.

7. Kompressionsartikel nach einem der voranstehenden Ansprüche, **dadurch gekennzeichnet, dass** die vordere Lage (v) und die hintere Lage (h) jeder Aufnahme (1) einen Strickfaden (4) enthält, der im Bereich der Spreizelemente (2) an Kreuzungsstellen (K) von einer Lage (v bzw. h) auf die gegenüberliegende Lage (h bzw. v) verkreuzt ist.

8. Kompressionsartikel nach Anspruch 7, **dadurch gekennzeichnet, dass** der Strickfaden (4) im Bereich der Spreizelemente (2) in einer in der Maschenreihe (m) zur Kreuzungsstelle (K) benachbarten Masche flott liegt und/oder dass der Strickfaden (4) einer Lage (v bzw. h) an den Kreuzungsstellen (K) als Masche (M) oder als Fanghenkel in der gegenüberliegenden Lage (h bzw. v) verkreuzt eingestrickt ist.

9. Kompressionsartikel nach einem der Ansprüche 7 oder 8, **dadurch gekennzeichnet, dass** sich die vordere Lage (v) mit der hinteren Lage (h) im Bereich der Spreizelemente (2) an den Kreuzungsstellen (K) vertauscht, und umgekehrt.

10. Kompressionsartikel nach einem der voranstehenden Ansprüche, **dadurch gekennzeichnet, dass** es sich um einen Handschuh handelt, wobei eine Aufnahme (1) zur Aufnahme eines Daumens und/oder wenigstens eine weitere Aufnahme (1a, 1b, 1c, 1d) zur Aufnahme eines Fingers dienen oder dass es sich um ein Zehenteil handelt, wobei wenigstens eine Aufnahme (1) zur Aufnahme einer Zehe und/oder weitere Aufnahmen (1a, 1b, 1c, 1d) zur Aufnahme weiterer Zehen dienen.

11. Kompressionsartikel nach einem der Ansprüche 4 bis 10, **dadurch gekennzeichnet, dass** das oder jedes Spreizelement (2) aus dem Strickfaden (4) des Grundgestricks (3) gestrickt ist, indem der Strickfaden (4) von einer Lage (v oder h) auf die gegenüberliegende Lage (h oder v) verkreuzt eingestrickt ist.

12. Kompressionsartikel nach einem der voranstehenden Ansprüche, wobei die Spreizelemente (2) ausgebildet sind, die in den Aufnahmen (1) aufnehmbaren Finger oder Zehen auf Abstand zueinander zu halten und/oder einen erhöhten Druck auf Zwischenräume zwischen den Fingern oder Zehen auszuüben.

13. Kompressionsartikel nach einem der voranstehenden Ansprüche, wobei sich das oder jedes Spreizelement (2) über mehr als die Hälfte der Länge der jeweils angrenzenden Aufnahme (1) erstreckt.

14. Verwendung eines Kompressionsartikels nach einem der voranstehenden Ansprüche in der Narbentherapie oder zur Behandlung von Verbrennungen, Verbrühungen oder plastisch-chirurgischen Rekonstruktionen einer Hand oder eines Fußes, oder zur postoperativen Kompressionstherapie im Bereich der plastisch-rekonstruktiven Chirurgie, wobei die Spreizelemente (2) beim Tragen des Kompressionsartikels an einer Hand oder einem Fuß auf die Zwischenräume der Finger oder Zehen einen erhöhten Druck ausüben und dadurch Narbenkeloide und/oder Narbenkontrakturen unterdrücken.

15. Verfahren zur Herstellung eines Kompressionsartikels nach einem der Ansprüche 1 bis 13, wobei der Kompressionsartikel nahtlos auf einer Flachstrickmaschine mit einem vorderen Nadelbett und einem diesem gegenüberliegenden hinteren Nadelbett gestrickt wird, wobei die vordere Lage (v) der Aufnahmen (1) mit dem vorderen Nadelbett und die hintere Lage (h) der Aufnahmen (1) mit dem hinteren Nadelbett gestrickt wird.

## Claims

1. Knitted compression article with a plurality of tubular or pocket-like receivers (1) for at least one finger or at least one toe of a wearer of the compression article, wherein a spacer element (2) is arranged at least between two adjacent receivers (1a, 1b), wherein each receiver (1) has a front layer (v) and a rear layer (h), wherein the front layer (v) and the rear layer (h) are seamlessly knitted to each other so as to form the tubular or pocket-like receiver (1), and in that the or each spacer element (2) is formed by connecting the front layer (v) and the rear layer (h) using knitting technology, **characterised in that** the or each spacer element (2) extends over at least 1/3 of the length of the respective adjoining receiver (1).

2. Compression article according to claim 1, **characterised in that** the compression article as a whole is seamless, wherein the spacer elements (2) are formed without seams.

3. Compression article according to one of the preceding claims, **characterised in that** the or each spacer element (2) is formed by crossing the front layer (v) and the rear layer (h).

4. Compression article according to one of the preceding claims, **characterised in that** the compression article has a basic knitted fabric (3) with an inner side (I) which can be turned towards the body extremity when worn and an outer side (A) opposite the latter, wherein the basic knitted fabric (3) is knitted along a stitch wale direction (s) extending at least substantially in the longitudinal direction (L) of the or each receiver (1) and has rows of stitches (m) running transversely to the stitch wale direction (s), wherein the basic knitted fabric (3) is a knit pattern or a purl pattern and comprises a front layer (v) and a rear layer (h) connected seamlessly to the front layer (v), wherein the front layer (v) and the rear layer (h) is formed from at least one elastic or inelastic knitting thread (4).

5. Compression article according to claim 4, **characterised in that** a weft thread (5) is incorporated or inserted into the basic knitted fabric (3).

6. Compression article according to one of the preceding claims, **characterised in** the spacer elements (2) extend in the direction of the rows of stitches (m) over at least two or three stitches.

7. Compression article according to one of the preceding claims, **characterised in that** the front layer (v) and the rear layer (h) of each receiver (1) contains a knitting thread (4) which in the region of the spacer elements (2) is crossed from one layer (v or h) to the opposite layer (h or v) at crossover points (K).

8. Compression article according to claim 7, **characterised in that** the knitting thread (4) in the region of the spacer elements (2) is floating in a stitch adjacent to the crossover point (K) in the row of stitches (m) and/or **in that** the knitting thread (4) of a layer (v or h) is cross-knitted in as a stitch (M) or as a tuck loop in the opposite layer (h or v) at the crossover points (K).

9. Compression article according to one of claims 7 or 8, **characterised in that** the front layer (v) and the rear layer (h) in the region of the spacer elements (2) are interchanged at the crossover points (K), and vice versa.

10. Compression article according to one of the preceding claims, **characterised in that** it is a glove, wherein a receiver (1) serves for receiving a thumb and/or at least one further receiver (1a, 1b, 1c, 1d) serves for receiving a finger or **in that** the compression article is a toe part, wherein at least one receiver (1) serves for receiving a toe and/or further receivers (1a, 1b, 1c, 1d) serve for receiving further toes.

11. Compression article according to one of claims 4 to 10, **characterised in that** the or each spacer element (2) is knitted from the knitting thread (4) of the basic knitted fabric (3) by cross-knitting in the knitting thread (4) from one layer (v or h) to the opposite layer (h or v).

12. Compression article according to one of the preceding claims, wherein the spacer elements (2) are formed to hold the fingers or toes which can be received in the receivers (1) at a distance from each other and/or to exert an increased pressure on gaps between the fingers or toes.

13. Compression article according to one of the preceding claims, wherein the or each spacer element (2) extends over more than half of the length of the respective adjoining receiver (1).

14. Use of a compression article according to one of the preceding claims in cicatricial treatment or for treating burns, scalds or reconstructions by plastic surgery of a hand or of a foot, or for post-operative compression treatment in the field of reconstructions by plastic surgery, wherein the spacer elements (2), when wearing the compression article on a hand or a foot, exert an increased pressure on the gaps between the fingers or toes and thus suppress cicatricial keloids and/or cicatricial contractions.

15. Method for producing a compression article according to one of claims 1 to 13, wherein the compression article is knitted to be seamless on a flat knitting machine having a front needle bed and a rear needle bed opposite the latter, wherein the front layer (v) of the receivers (1) is knitted using the front needle bed and the rear layer (h) of the receivers (1) is knitted using the rear needle bed.

## Revendications

1. Article de contention tricoté avec une multitude de logements (1) de forme tubulaire ou en forme de compartiment pour au moins un doigt ou au moins un orteil d'une personne portant l'article de contention, dans lequel un élément d'écartement (2) est disposé au moins entre deux logements (1a, 1b) adjacents, dans lequel chaque logement (1) présente une couche avant (v) et une couche arrière (h), dans lequel la couche avant (v) et la couche arrière (h) sont tricotées sans couture l'une avec l'autre pour réaliser le logement (1) de forme tubulaire ou en forme de compartiment, et que l'élément d'écartement ou chaque élément d'écartement (2) est formé par une liaison technique de tricotage de la couche avant (v) et de la couche arrière (h), **caractérisé en ce que** l'élément d'écartement ou chaque élément d'écartement (2) s'étend sur au moins 1/3 de la longueur du logement (1) respectivement adjacent.

2. Article de contention selon la revendication 1, **caractérisé en ce que** l'article de contention est globalement sans couture, dans lequel les éléments d'écartement (2) sont réalisés sans coutures.

3. Article de contention selon l'une quelconque des revendications précédentes, **caractérisé en ce que** l'élément d'écartement ou chaque élément d'écartement (2) est formé par le croisement de la couche avant (v) et de la couche arrière (h).

4. Article de contention selon l'une quelconque des revendications précédentes, **caractérisé en ce que** l'article de contention présente un tricot principal (3) avec un côté intérieur (I) pouvant être tourné vers l'extrémité corporelle lors du port et un côté extérieur (A) faisant face à celui-ci, dans lequel le tricot principal (3) est tricoté le long d'une direction de colonnes de mailles (s) s'étendant au moins sensiblement dans le sens longitudinal (L) du ou de chaque logement (1) et présente des rangées de mailles (m) s'étendant de manière transversale par rapport à la direction de colonnes de mailles (s), dans lequel le tricot principal (3) comprend un tricot droite-gauche ou un tricot droite-droite et une couche avant (v) ainsi qu'une couche arrière (h) reliée sans couture à la couche avant (v), dans lequel la couche avant (v) et la couche arrière (h) sont formées à partir d'au moins un fil de tricotage (4) élastique ou non élastique.

5. Article de contention selon la revendication 4, **caractérisé en ce qu'**un fil de trame (5) est intégré ou placé dans le tricot principal (3).

6. Article de contention selon l'une quelconque des revendications précédentes, **caractérisé en ce que** les éléments d'écartement (2) s'étendent en direction des rangées de mailles (m) sur au moins deux ou trois mailles.

7. Article de contention selon l'une quelconque des revendications précédentes, **caractérisé en ce que** la couche avant (v) et la couche arrière (j) de chaque logement (1) contiennent un fil de tricotage (4), qui est croisé dans la zone des éléments d'écartement (2) sur des emplacements de croisement (K) d'une couche (v ou h) sur la couche faisant face (h ou v).

8. Article de contention selon la revendication 7, **caractérisé en ce que** le fil de tricotage (4) se situe de manière flottante dans la zone des éléments d'écartement (2) dans une maille adjacente dans la rangée de mailles (m) de l'emplacement de croisement (K), et/ou que le fil de tricotage (4) d'une couche (v ou h) est tricoté de manière croisée en tant que maille (M) ou en tant que flotté dans la couche (h ou v) faisant face sur les emplacements de croisement (K).

9. Article de contention selon l'une quelconque des revendications 7 ou 8, **caractérisé en ce que** la couche avant (v) permute avec la couche arrière (h) dans la zone des éléments d'écartement (2) sur les emplacements de croisement (K), et inversement.

10. Article de contention selon l'une quelconque des revendications précédentes, **caractérisé en ce qu'**il s'agit d'un gant, dans lequel un logement (1) sert à loger un pouce et/ou au moins un autre logement (1a, 1b, 1c, 1d) sert à loger un doigt, ou qu'il s'agit d'une partie d'orteil, dans lequel au moins un logement (1) sert à loger un orteil et/ou d'autres logements (1a, 1b, 1c, 1d) servent à loger d'autres orteils.

11. Article de contention selon l'une quelconque des revendications 4 à 10, **caractérisé en ce que** l'élément d'écartement ou chaque élément d'écartement (2) est tricoté à partir du fil de tricotage (4) du tricot principal (3) **en ce que** le fil de tricotage (4) est tricoté de manière croisée depuis une couche (v ou h) sur la couche (h ou v) faisant face.

12. Article de contention selon l'une quelconque des revendications précédentes, dans lequel les éléments d'écartement (2) sont réalisés pour maintenir les doigts ou orteils pouvant être logés dans les logements (1) à distance les uns par rapport aux autres et/ou pour exercer une pression plus élevée sur des espaces intermédiaires entre les doigts ou orteils.

13. Article de contention selon l'une quelconque des revendications précédentes, dans lequel l'élément d'écartement ou chaque élément d'écartement (2) s'étend sur plus de la moitié de la longueur du logement (1) respectivement adjacent.

14. Utilisation d'un article de contention selon l'une quelconque des revendications précédentes dans la thérapie de cicatrices ou pour le traitement de brûlures, d'ébouillantages ou de reconstructions en chirurgie plastique d'une main ou d'un pied, ou pour la thérapie par contention postopératoire dans le domaine de la chirurgie de reconstruction plastique, dans laquelle les éléments d'écartement (2) exercent une pression plus élevée, lorsque l'article de contention est porté sur une main ou un pied, sur les espaces intermédiaires des doigts ou orteils et éliminent ce faisant les cicatrices chéloïdes et/ou les brides.

15. Procédé de fabrication d'un article de contention selon l'une quelconque des revendications 1 à 13, dans lequel l'article de contention est tricoté sans couture sur un métier à tricoter à plat avec un lit d'aiguilles avant et un lit d'aiguilles arrière lui faisant face, dans lequel la couche avant (v) des logements (1) est tricotée avec le lit d'aiguilles avant et la couche arrière (h) des logements (1) est tricotée avec le lit d'aiguilles arrière.
